# DEMANDE DE BREVET EUROPEEN

(11) **EP 0 567 378 A1**
(43) Date de publication de la demande: **27.10.1993**
(21) Numéro de dépôt: 93400997.8
(22) Date de dépôt: 16.04.1993
(51) Int. Cl.: G02F 1/35, C07D 307/54

(54) **Matériaux photoréticulables, utilisables comme sources laser vertes ou bleues et doubleur de fréquence comprenant ces matériaux photoréticulables**

(30) Priorité: 22.04.1992 FR 9204924
(71) Demandeur: THOMSON-CSF, 75008 Paris (FR)
(72) Inventeur: Le Barny, Pierre, F-92402 Courbevoie Cédex (FR); Muller, Sophie, F-92402 Courbevoie Cédex (FR); Lemoine, Vincent, F-92402 Courbevoie Cédex (FR); Dubois, Jean-Claude, F-92402 Courbevoie Cédex (FR)
(74) Mandataire: Guérin, Michel

(57) **Abrégé**

L' invention concerne des matériaux composites transparents dans le visible et actifs en optique non linéaire. Ces matériaux comprennent un polymère comportant un groupement photoréticulable, et une petite molécule active possédant au moins deux fonctions photoréticulables identiques à celles du polymère. Ces matériaux offrent l'intérêt d'être particulièrement stables dans le temps.

L'invention concerne également un doubleur de fréquence utilisant ces matériaux et permettant l'obtention de sources laser vertes ou bleues.

## Description

La présente invention concerne des matériaux photoréticulables actifs en optique non linéaire et transparents dans le visible. Elle concerne également l'utilisation de ces matériaux comme sources laser vertes ou bleues ainsi qu'un doubleur de fréquence comprenant comme matériau actif un matériau photoréticulable selon l'invention.

Plus précisément, l'invention concerne un nouveau système formé d'un polymère ayant dans sa chaîne latérale un groupement photoréticulable et d'une petite molécule comprenant un groupement chromophore sur lequel sont attachés au moins deux groupements photoréticulables identiques au groupement photoréticulable du polymère. Le groupement chromophore, orienté au moyen d'un champ électrique continu donne naissance à des effets non linéaires du second ordre en optique.Le groupement réticulable sous l'action de la lumière ultraviolette permet de geler, au cours de la polarisation du matériau, l'orientation des entités responsables des effets quadratiques en optique. L'invention concerne également la possibilité d'accéder à de nouvelles sources laser vertes ou bleues intégrées, par doublage de fréquence de diodes laser émettant entre entre 0,8 et 1 µm, en réalisant aisément un quasi accord de phase par inversion périodique de l'orientation des chromophores non linéaires grâce au matériau proposé par la demanderesse.

L'une des voies les plus prometteuses pour l'obtention de sources laser vertes ou bleues semble être celle qui utilise le doublement de fréquence de diodes laser émettant dans le proche infra-rouge. Cela est réalisé en utilisant un matériau possédant une susceptibilité d'ordre deux χ⁽²⁾ non nulle et suffisamment transparent dans le visible pour empêcher l'absorption du second harmonique lors de sa propagation dans le milieu non linéaire. A ce stade deux types de matériaux peuvent remplir ces conditions:
- Les matériaux inorganiques comme le niobate de lithium ou le KTP.
- Et les matériaux organiques.

Mais les matériaux organiques possèdent des avantages décisifs sur les matériaux inorganiques. En effet,
- Ils ont des temps de réponse très courts car les effets non linéaires auxquels ils donnent naissance sont d'origine purement électronique.
- Ils présentent un seuil de dommage optique plus élevé que les matériaux inorganiques.
- Enfin il est possible de faire varier très facilement la structure chimique des composés organiques, ce qui permet d'ajuster en particulier la transparence et la valeur de l'hyperpolarisabilité de ces molécules, ces deux paramètres variant en sens inverse.

Cependant, quel que soit le type de matériau envisagé le problème majeur des doubleurs de fréquence réside dans l'accord de phase à réaliser impérativement entre l'onde électromagnétique et l'onde harmonique créée. En effet l'onde harmonique créée se propage dans un matériau avec un vecteur d'onde k2, tel que: 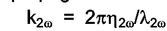
où η_{2ω} est l'indice de réfraction du matériau à la fréquence 2m, si ω est la fréquence de l'onde électromagnétique incidente qui se propage avec un vecteur k_{ω} tel que kₘ = 2πη_{ω} /λ_{ω}. η_{ω} étant l'indice de réfraction du matériau à la fréquence m.

Généralement η_{ω} est différent de η2_{ω}, et le déphasage existant entre l'onde incidente et l'onde harmonique limite de faτon rédhibitoire l'efficacité de conversion de la fréquence ω en 2m. On appelle longueur de cohérence Lc, la longueur du matériau au bout de laquelle ce déphasage atteint π, Lc étant donc définie de la manière suivante : Lc (k2m - 2kω)=π, soit encore Lc = λ_{ω}/4 (η2_{ω}- η_{ω}).

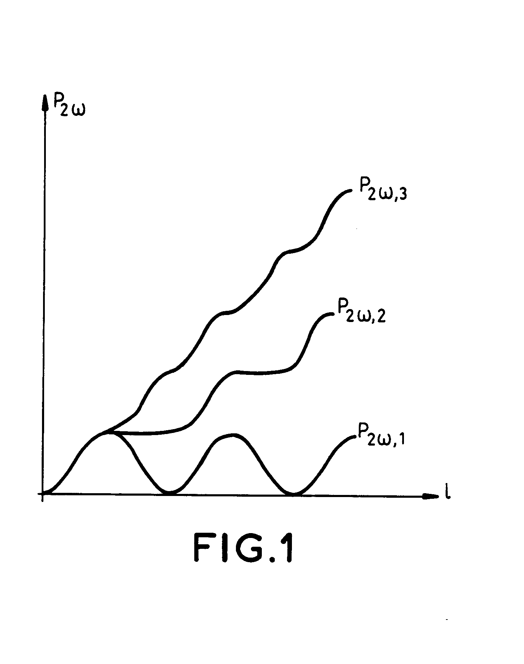

Une solution connue pour pallier ce problème consiste à utiliser une perturbation périodique avec un pas A de manière à former un réseau de susceptibilité non linéaire d'ordre 2 notée χ⁽²⁾ responsable de la génération de seconde harmonique. En choisissant astucieusement A tel que Lc = A/2 on parvient à cumuler la puissance émise à la fréquence 2m au cours du chemin parcouru par l'onde électromagnétique incidente comme l'illustre la figure 1, représentant l'évolution de la puissance créée à la fréquence 2m, en fonction de la longueur 1 parcourue au sein du doubleur. Sans réseau on obtient la courbe P_{2ω,1} en fonction de 1, en utilisant une structure réseau dans laquelle on crée périodiquement des domaines dans lesquels le coefficient χ⁽²⁾n'est pas nul, séparés de domaines dans lesquels le coefficient χ⁽²⁾ est nul, on obtient la courbe P_{2ω,2}. Enfin en créant un réseau de domaines +χ⁽²⁾ séparés de domaines -χ⁽²⁾ on obtient la courbe P2m,3 , la plus performante.

La présente invention propose des matériaux organiques au sein desquels on est capable de réaliser un réseau de domaines +χ⁽²⁾ séparés par des domaines -χ⁽²⁾ permettant la réalisation de doubleur de fréquence organique très performant.

Il s'agit de matériaux polymères très prometteurs dans le domaine de l'optique intégrée puisqu'ils peuvent se déposer facilement en couche mince sur de grandes surfaces,ce qui les rend compatibles avec la technologie semi-conducteur. De plus, les films ainsi obtenus sont de très bonne qualité optique (seuil de dommage optique élevé, faible diffusion ...). La non centrosymétrie de ces matériaux nécessaire à l'obtention des effets quadratiques en optique est obtenue par application d'un champ électrique continu intense à une température voisine de la température de transition vitreuse du polymère ,suivie d'un refroidissement à température ambiante sous champ.

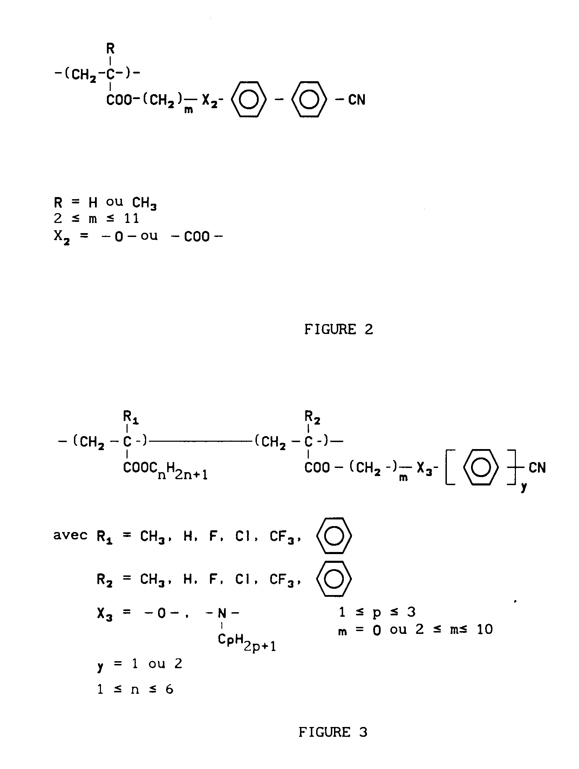

Jusqu'à présent, les études effectuées dans le domaine des matériaux organiques utilisables en optique non linéaire ont surtout porté sur les matériaux présentant une valeur élevée de susceptibilité X2 ce qui conduit à choisir des structures chimiques pour les chromophores possédant une forte hyperpolarisabilité. Ce choix est fait aux dépens de la transparence du matériau dans le domaine des longueurs d'onde du visible. Néanmoins, selon l'art antérieur, des homopolymères dont la structure chimique est représentée figure 2, présentant un maximum d'absorption en dessous de 350 nm ont été synthétisés (Eur.Polym.J.18 651 (1982)) Ces matériaux possèdent une susceptibilité du second ordre suffisante pour envisager des applications en génération de seconde harmonique. Cependant ces polymères ont une faible valeur de température de transition vitreuse qui favorise les problèmes de relaxation des chaînes latérales après orientation et par ailleurs donnent naissance à des mésophases smectiques ou nématiques qui induisent des phénomènes de diffusion de la lumière rédhibitoire dans les applications en optique guidée. Des polymères amorphes transparents dans le visible ayant la structure présentée Figure 3 ont également été proposé par la demanderesse (Brevet 90 08233) Ces polymères ont des propriétés intéressantes en optique non linéaire néanmoins la stabilité de ces dernières est limitée dans le temps. De plus aucun de ces matériaux ne permet la réalisation d'une structure composée de microdomaines juxtaposés présentant une polarisation alternée, nécessaire à l'obtention de l'accord de phase.

La voie la plus prometteuse pour pallier les problèmes de relaxation semble être celle qui incorpore le chromophore dans une macromolécule tridimensionnelle de manière telle que les points de réticulation fassent partie du chromophore lui-même. Une solution a été proposée pour obtenir un tel réseau tridimensionnel :

-La réticulation thermique

La réticulation thermique met en jeu soit des petites molécules actives en optique non linéaire possédant au moins deux fonctions susceptibles de réagir sous l'action de la chaleur avec d'autres molécules polyfonc- tionnelles, pas nécessairement porteuses de chromophores (M. Eich et al, J. Appl. Phys., 66 7 (1989); D. Jungbauer et al, Appl. Phys. Letter, 56 26 (1990); P. Le Barny et al, brevet 91 08233); soit des polymères non linéaires à chaînes latérales portant des fonctions réticulables par voie thermique(S. Muller et al. Brevet 90 13041).

La réticulation thermique n'est pas adaptée à l'obtention de microdomaines dont le sens de la polarisation est alterné puisqu'il est impossible de polariser sélectivement des zones précises du film sans réticuler l'ensemble de ce dernier ce qui empêche toute orientation en sens opposé ultérieure.

Afin de répondre aux critères suivants:
- Structure chimique du matériau ayant un maximum d'absorption inférieur à 350 nm pour les sources laser vertes ou 300 nm pour les sources laser bleues
- Films réalisés à partir de ces matériaux de bonne tenue mécanique et de bonne qualité optique.
- Obtention aisée de la juxtaposition de domaines ayant des sens de polarisation opposés.

La présente invention propose un ensemble de matériaux formés d'un polymère porteur de fonctions photoréticulables et d'une petite molécule active en optique non linéaire possédant au moins deux fonctions photoréticulables, lesdites fonctions étant identiques à la fonction photoréticulable du polymère.

La présente invention propose également un doubleur de fréquence comprenant une couche de matériau selon l'invention dans laquelle est inscrit un réseau constitué de domainer D₁ de susceptibilité non linéaire d'ordre 2 +_{X}(²) et de domaines D₂ de susceptibilité non linéaire d'ordre 2 -χ⁽²⁾, les domaines Dₗet D₂ étant obtenus successivement par photoréticulation du matériau et en présence respectivement d'un champ électrique + E et d'un champ électrique - E.

Le doubleur de fréquence selon l'invention peut avantageusement être réalisé à partir d'un matériau transparent dans le visible, pouvant doubler la fréquence d'une diode laser émettant dans la gamme 0,8 - 1 f..lm de manière à constituer une source laser verte ou bleue.

Plus précisément, l'invention concerne un matériau composite utilisable pour des sources laser vertes où bleues comprenant un polymère amorphe constitué d'un squelette sur lequel sont rattachées des chaînes latérales et une petite molécule capable de générer des effets non linéaires en optique non linéaire, caractérisé en ce que le polymère comprend dans sa chaîne latérale une fonction photoréticulable séparée du squelette par un groupement intermédiaire, la petite molécule possède au moins deux fonctions photoréticulables identiques à la fonction photoréticulable du polymère et lesdits polymère et petite molécule présentent un maximum d'absorption inférieur 350 nm pour les sources laservertes et inférieur 300 nm pour les sources laser bleues.

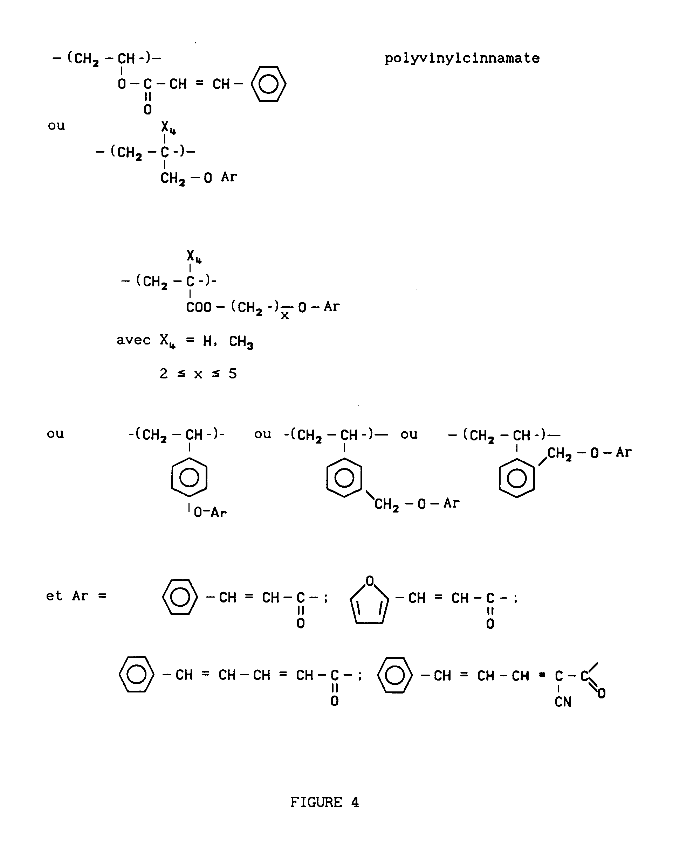

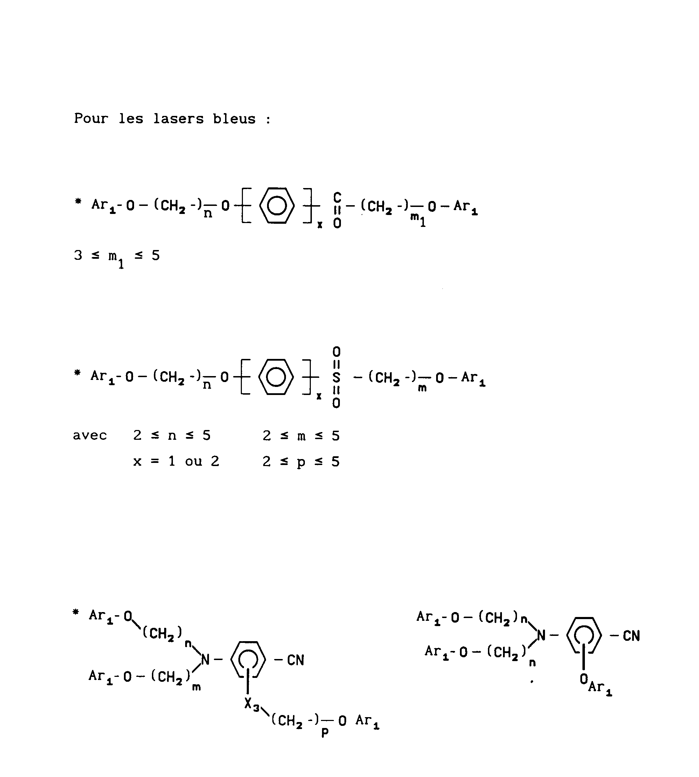

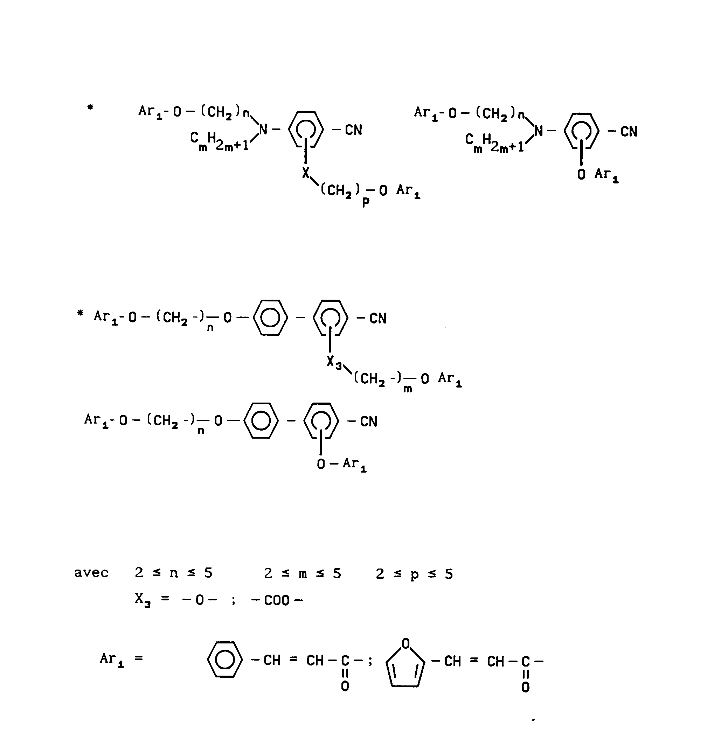

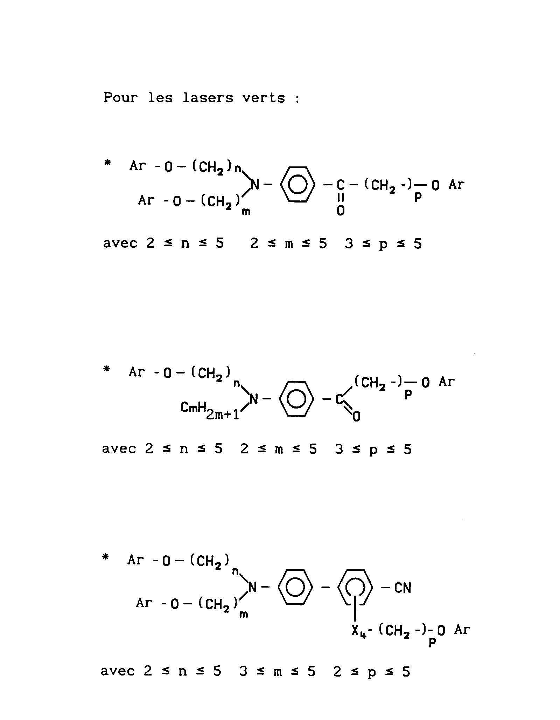

De préférence, les matériaux selon l'invention utilisent les polymères illustrés à la figure 4.

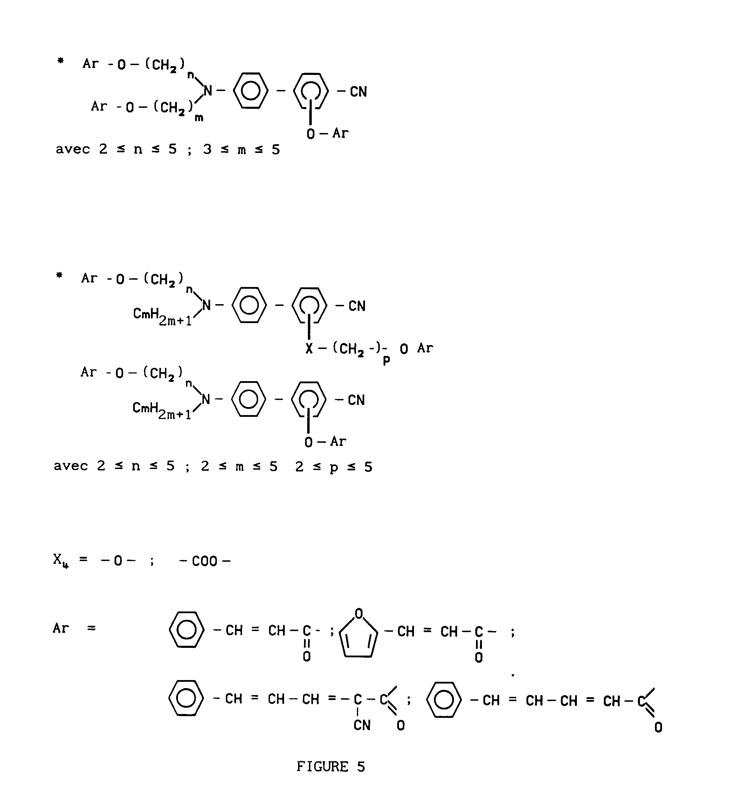

De préférence, ces matériaux utilisent les petites molécules de la figure 5.

La présente invention a enfin pour objet le procédé de synthèse du 4 furylacryloyloxyethoxy) 4' (furylacryloyloxybutanoyle) biphenyle, petite molécule utilisée dans les matériaux selon l'invention.

L'invention sera mieux comprise et d'autres avantages apparaîtront à la lecture de la description qui va suivre donnée à titre non limitatif et grâce aux figures annexées parmi lesquelles:
- la figure 1 illustre le comportement de la puissance créée à fréquence 2m sous l'action d'une onde électromagnétique en fonction du chemin parcouru au sein d'un doubleur de fréquence ;
- Les figures 2 et 3 représentent la structure moléculaire des homopolymères cités précédemment, selon l'art antérieur;
- La figure 4 représente la structure moléculaire des homopolymères hôtes selon l'invention ;
- La figure 5 représente la structure chimique des petites molécules actives en optique non linéaire photoréticulables ;

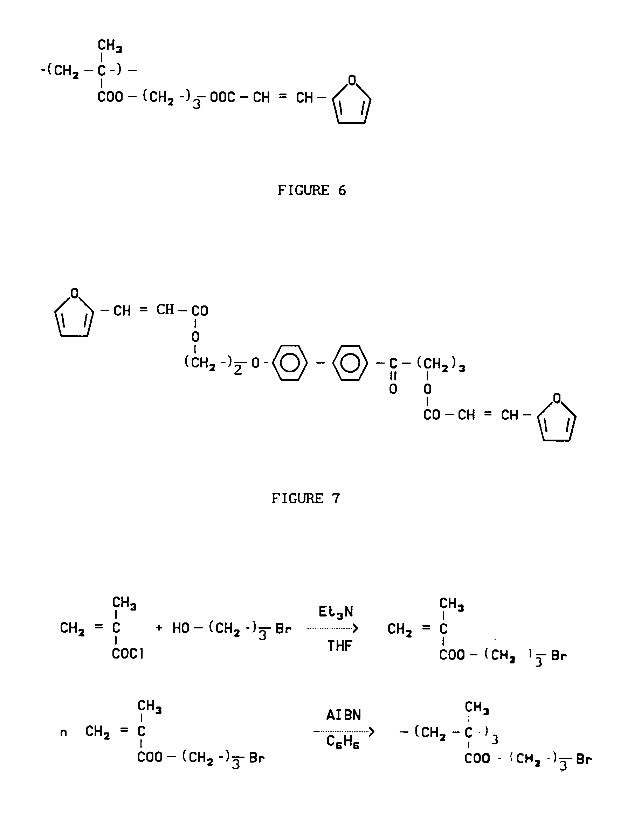

- La figure 6 représente la structure chimique d'un exemple d'homopolymère selon l'invention ;
- La figure 7 représente la structure chimique d'un exemple de petite molécule active en optique non linéaire photoréticulable ;
- La figure 8 représente le schéma réactionnel de synthèse de l'homopolymère de l'exemple illustré à la figure 6 ;
- La figure 9 représente le schéma réactionnel de synthèse de la petite molécule photoréticulable de l'exemple illustré à la figure 7 ;

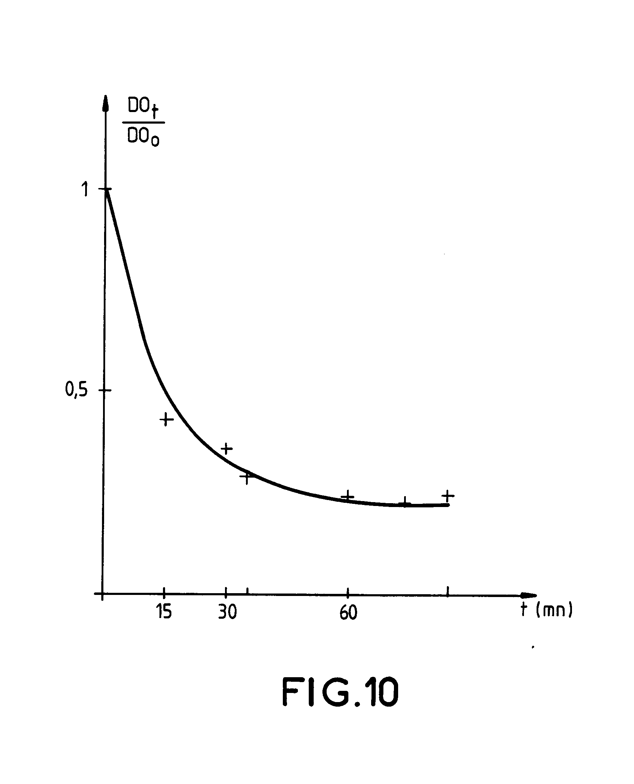

- La figure 10 donne l'évolution de la densité optique d'un système polymère petite molécule selon l'invention en fonction du temps d'irradiation à 312 nm.

### EXEMPLE

L'exemple concerne la synthèse du poly (méthacryloyloxypropylfurylacrylate) et du 4 (furylacryloy- loxyethyloxy) 4' (furylacryloyloxybutanoyle) biphényle.

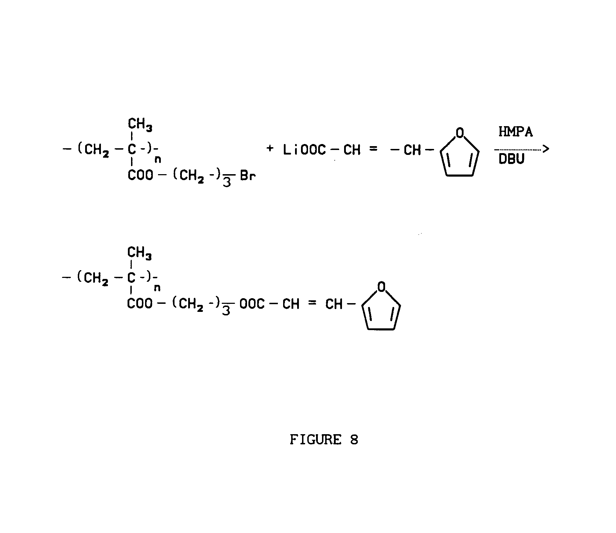

Synthèse de l'homopolymère:
L 'homopolymère est préparé en 3 étapes à partir du chlorure de méthacryloyle et du 3 bromo propanol commerciaux selon le schéma réactionnel de la figure 8.

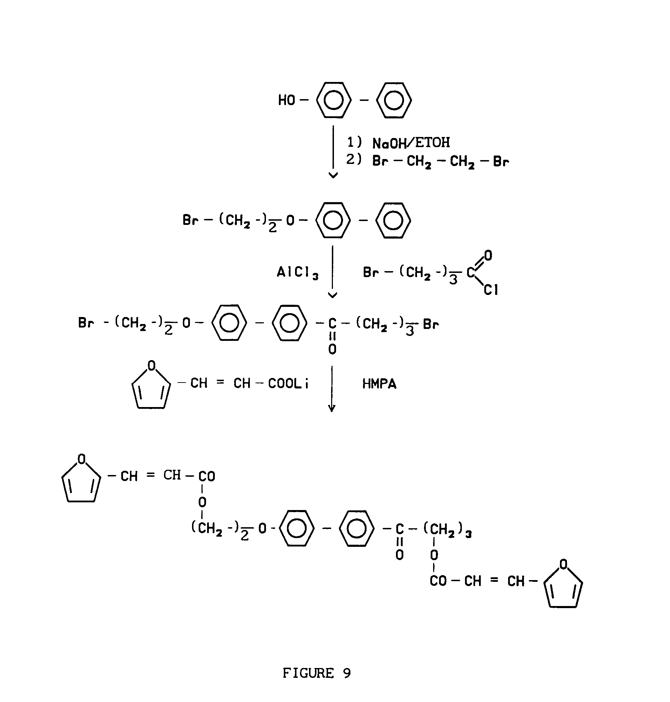

Synthèse de la petite molécule:
La molécule active en optique non linéaire et photoréticulable est obtenue en 5 étapes à partir de l'hydroxy biphényle selon le schéma réactionnel de la figure 9.

Synthèse du 4(2bromo éthoxy) biphényle :
Dans un erlenmeyer de 250 ml, on introduit 150 ml d'éthanol et 3,38g de sodium. On chauffe à reflux jusqu'à disparition du sodium, puis on ajoute 25g (0,147 mole) de 4 hydroxy biphényle. On chauffe de nouveau à reflux pour former le phénate correspondant. On additionne 110g de 1,2 dibromo éthane et on continue de chauffer 16h à reflux. Le milieu réactionnel est coulé dans 21 d'eau. Le produit brut précipite. On le sépare par filtration et on le sèche sous vide. L'éther est extrait du solide par lavage avec du cyclohexane à ébullition. Le solide ainsi obtenu après évaporation du cyclohexane est purifié par chromatographie sur gel de silice (éluant: CH₂CI₂) suivie d'une recristallisation dans l'hexane. On obtient 9,98g de produit pur. Rendement: 24,5%.

### Point de fusion: 112°C.

Synthèse du chlorure de 4 bromobutanoyle:
Dans un erlenmeyer de 250ml on introduit 30g d'acide 4 bromo butanoique, 26g de chlorure de thionyle distillé et 22ml de benzène . On chauffe à reflux pendant 3h. On évapore le solvant et le chlorure de thionyle en excès sous vide de l'évaporateur et le produit brut ainsi obtenu est purifié par distillation sous vide..On obtient 21,69g de chlorure pur.

### Rendement: 65,1%.

Synthèse du 4(4 bromo butanoyle) 4' ( 2 bromoethoxy) biphényle :
Dans un tricol de 250ml on met en suspension 7,7g de chlorure d'aluminium dans 80ml de 1,1,2,2 tétra- chloroéthane. On ajoute goutte à goutte10,7g de chlorure de 4 bromo butanoyle à T ambiante. On chauffe à 52°C pendant 20mn. Il se forme un complexe à base de chlorure d'aluminium On refroidit la solution de complexe ainsi formée à 14°C et on ajoute goutte à goutte une solution de 16g de 4( 2 bromo éthoxy) biphényle dans 80ml de solvant, dans le milieu réactionnel. On laisse agiter 1 h à 14°C puis on revient à T ambiante et on chauffe à 50°C pendant 1 h. Ace stade, on ajoute après avoir refroidi à14°C le milieu réactionnel, un nouveau lot de complexe formé à partir de 3,85g de AICI3 et 5,35g de chlorure de 4 bromo butanoyle. On agite de nouveau pendant 1 h à 14°C et on chauffe 3h à 50°C.

Le milieu réactionnel est ensuite coulé dans l'eau acidifié par HCI. On extrait à l'éther le produit brut. Celui- ci est purifié par chromatographie sur gel de silice ( éluant: toluène) suivie d'une recristallisation dans le cyclohexane. On obtient 10,62g de cétone pure . Rendement: 43,2%.

### Point de fusion: 103°C

Synthèse du furylacrylate de lithium.

Dans un erlenmeyer de 250ml on dissout 2,73g d'hydroxyde de lithium dans 40,5ml de méthanol. On ajoute 17,13g d'acide furylacrylique et on agite à T ambiante pendant 1 h. Il subsiste un insoluble qu'on élimine par filtration. Le filtrat est coulé goutte à goutte dans 1,81 d'éther. Le sel précipite. On obtient 14,45g de furylacrylate de lithium.

Rendement: 88,8%.

Synthèse du 4 (furylacryloyloxyethoxy) 4'(furylacryloyloxybutanoyle) biphényle:
Dans un erlenmeyer de 100 ml on dissout 5g du dérivé di bromé précédent puis on introduit 4,2 g de furylacrylate de lithium.On agite en chauffant à 50°C pendant 16h. Le milieu réactionnel est coulé dans l'eau. Le produit brut précipite. Il est purifié par chromatographie sur gel de silice (éluant :CH₂CI₂)suivie d'une recristallisation dans un mélange chloroformeéthanol. On obtient 4,7g de di-ester pur.
Rendement : 74 %, Point de fusion 124 °C

### OBTENTION DE FILMS ORIENTES ET RETICULES

On réalise une solution de polymère hôte et de petite molécule active en optique non linéaire de telle manière que la concentration en chaînes latérales du polymère porteuses des fonctions phoréticulables soit égale à au moins 2 fois celle du chromophore. Le mélange est déposé par centrifugation sur un substrat conducteur ou semiconducteur. Le film obtenu est séché sous vide. Après élimination du solvant d'enduction, le film est polarisé par application d'un champ électrique au moyen d'une pointe Corona portée à un potentiel supérieur ou égal à 4kV. Parallèlement le film est irradié au moyen d'une lampe ou d'un ensemble de lampes émettant dans la bande d'absorption du groupement photoréticulable pendant le temps nécessaire à la photoréticulation. Ce temps ayant été déterminé au préalable par une étude spectrométrique.

A titre d'exemple une solution à 50g/1 de poly(méthacryloyoxypropylfurylacrylate) et à 20g/1 de 4(furyla- cryloyloxyéthyloxy) 4' (furylacryloyloxybutanoyle) biphényle dans le chloroforme est tout d'abord déposée par centrifugation ( 2500 tours par minute pendant 30secondes) sur un substrat en quartz afin de réaliser l'étude spectrométrique. Le film ayant un maximum d'absorption à 301 nm, une lampe émettant à 312 nm est choisie pour effectuer la photoréticulation. La figure 9 montre l'évolution de la densité optique mesurée au maximum d'absorption, en fonction du temps. On constate une rapide décroissance de la densité optique dans les 15 premières minutes d'irradiation en accord avec la réticulation du système. On peut estimer que l'état d'avancement de la réaction est suffisant au bout de 60mn.

Compte-tenu de ces résultats il est maintenant possible d'obtenir un film réticulé et polarisé. Pour ce faire on dépose un nouveau film sur un substrat conducteur ou semiconducteur, dans les mêmes conditions que précédemment et on irradie a 312nm en même temps qu'on applique un champ continu moyen d'une pointe Corona portée à 4kV.

### OBTENTION DE LA STRUCTURE ALTERNEE

Le mélange polymère-petite molécule active en optique non linéaire est déposé par centrifugation sur un ensemble couche tampon-substrat conducteur.

En effet, une couche organique tampon d'indice inférieur à celui du matériau selon l'invention est préalablement déposée sur le substrat pour assurer le confinement de la lumière à guider au sein de la couche active, pour la réalisation d'un doubleur de fréquence. L'irradion nécessaire à la réticulation effectuée autour de 300 nm peut être avantageusement réalisée par interférence de deux faisceaux faisant un angle 0 de part et d'autre de la normale au plan des couches. Le contrôle de l'angle 0 permet d'ajuster le pas du réseau et d'irradier dans un premier temps seules certaines zones en présence d'un champ électrique + E créé par une décharge Corona de manière à définir les domaines D₁ de susceptibilité non linéaire +χ⁽²⁾.

Dans un second temps, l'opération d'irradiation est renouvelée en ayant soin de décaler d'une longueur de cohérence les interférences constructives et ce en présence d'un champ électrique opposé.

On obtient ainsi un film de matériau actif constitué de domaines alternés de susceptibilité non linéaire +χ⁽²⁾, et -_{X}(²),transparent dans le visible et capable de doubler une fréquence correspondant à des longueurs d'onde comprises entre 0,8 et 1 µm.

## Revendications

1. Matériau utilisable pour des sources laser vertes ou bleues comprenant un polymère amorphe constitué d'un squelette sur lequel sont rattachées des chaînes latérales et une petite molécule capable de générer des effets non linéaires en optique non linéaire, caractérisé en ce que le polymère comprend dans sa chaîne latérale une fonction photoréticulable séparée du squelette par un groupement intermédiaire, la petite molécule possède au moins deux fonctions photoréticulables identiques à la fonction photoréticulable du polymère et lesdits polymère et petite molécule présentent un maximum d'absorption inférieur à 350 nm pour les sources laser vertes et inférieur à 300 nm pour les sources laser bleues.

2. Matériau selon la revendication 1, caractérisé en ce que le polymère répond à l'une des formules chimiques suivantes : 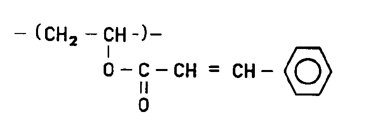
polyvinylcinnamate ou 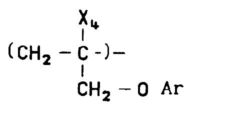 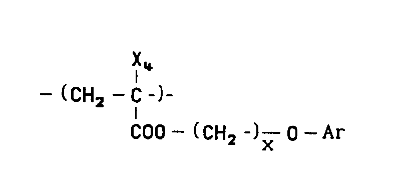
avec X₄=H,CH₃ 2≦x≦5 ou 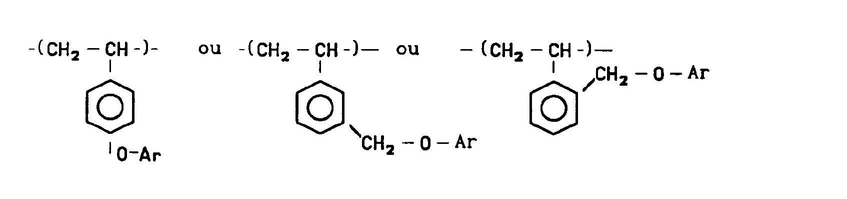
et Ar = 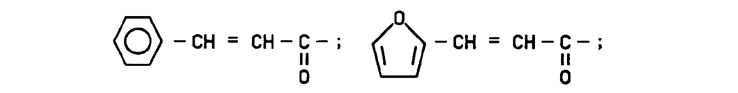 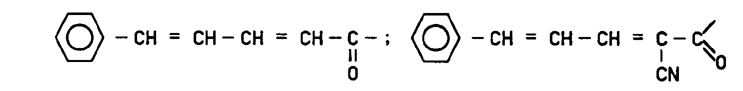
Matériau selon l'une des revendications 1 ou 2, caractérisé en ce que la petite molécule répond à l'une des formules chimiques suivantes : 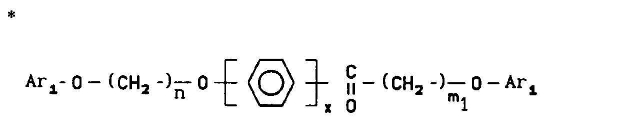
3≦m₁≦5 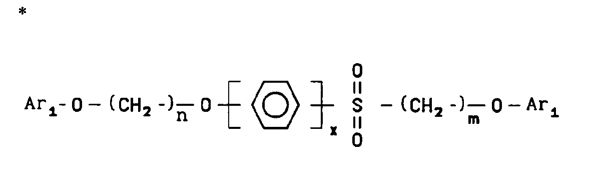
avec
2≦n≦52≦m≦5
x=1ou2 2≦p≦5 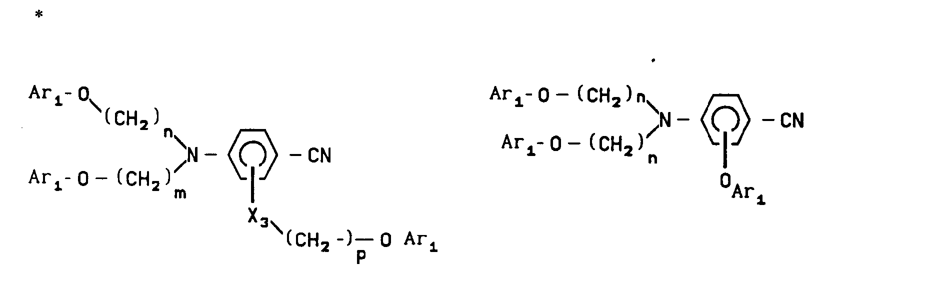 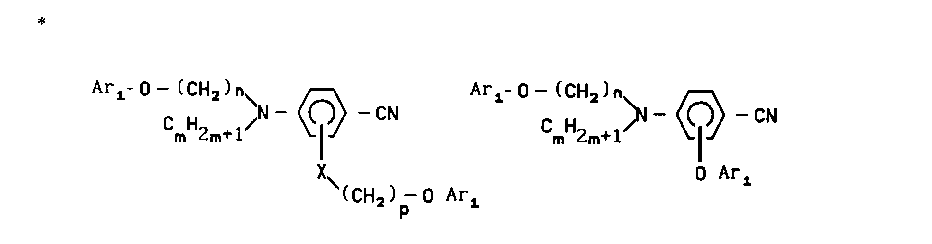 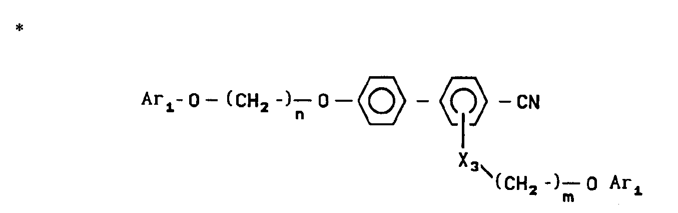 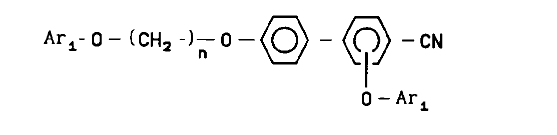
avec
2≦n≦5 2≦m≦5 2≦p≦5
X₃ = -O-; -COO-Ar₁ =

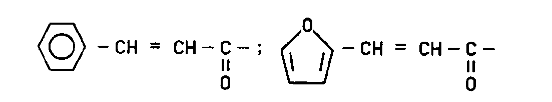

4. Matériau selon l'une des revendications 1 ou 2, caractérisé en ce que la petite molécule répond à l'une des formules chimiques suivantes : 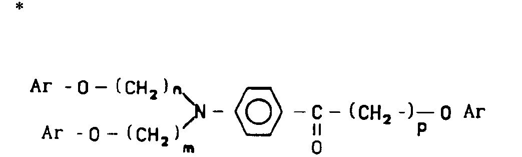
avec 2≦n≦5 2≦m≦5 2≦p≦5 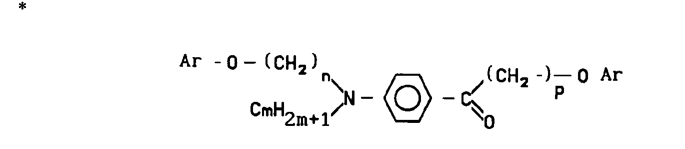
avec 2≦n≦5 2≦m≦5 3≦p≦5 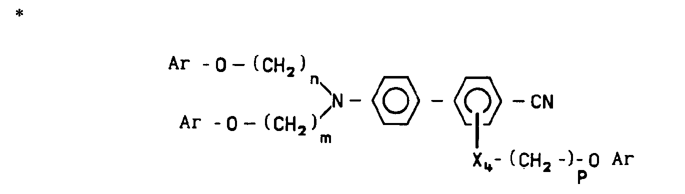
avec 2≦n≦5 3≦m≦5 2≦p≦5 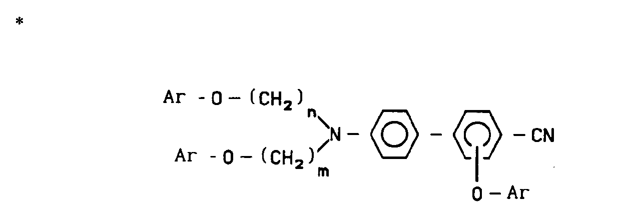
avec 2≦n≦5;3≦m≦5 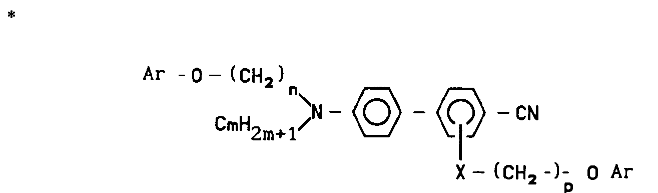 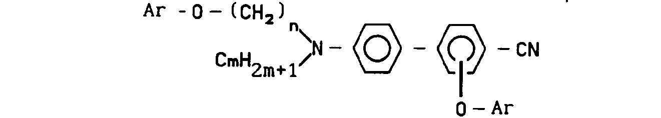
avec 2≦n≦5; 2≦m≦5 2≦p≦5 X_{4 =} -O-; -COO-Ar =

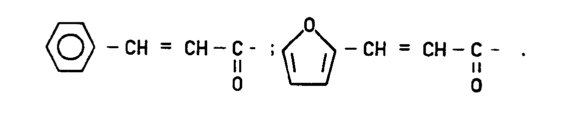

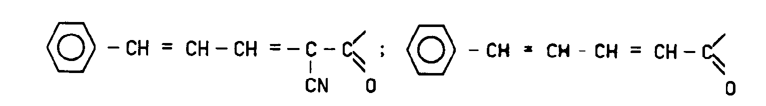

5. Doubleur de fréquence caractérisé en ce qu'il comprend une couche de matériau selon la revendication 1 dans laquelle est inscrit un réseau constitué de domaines D₁ de susceptibilité non linéaire d'ordre 2 +χ⁽²⁾ et de domaines D₂ de susceptibilité non linéaire d'ordre 2 -χ⁽²⁾, les domaines D₁ et D₂étant obtenus successivement par photoréticulation du matériau et en présence respectivement d'un champ électrique + E et d'un champ électrique - E.

6. Procédé de synthèse du 4 (furylacryloyloxyethoxy) 4' (furylacryloyloxybutanoyle) biphenyle caractérisé en ce qu'il comporte les étapes suivantes :
- O alkylation de l'hydroxybiphenyle en présence de 1,2 dibromoéthane et d'ethanol et obtention du 4 (2 bromo éthoxy) biphenyle ;
- Synthèse du chlorure de 4 bromobutanoyle en présence d'acide bromo butanoïque, de chlorure de thionyle et de benzène ;
- Synthèse du 4 (4 bromo butanoyle) 4' (2 bromoethoxy) biphenyl par réaction de Friedel et Craft du chlorure de 4 bromobutanoyle en présence de chlorure d'aluminium ;
- Synthèse du furylacrylate de lithium à partir d'hydroxyde de lithium, de methanol et d'acide furylacrylique ;
- Esterification du 4 (4 bromobutanoyle) 4' (2 bromoethoxy) biphenyl en présence du furylacrylate de lithium et l'hexamethylphosphortriamide (HMPA).
